# EUROPEAN PATENT APPLICATION

(11) **EP 4 471 012 A1**
(43) Date of publication of application: **04.12.2024**
(21) Application number: 23746369.0
(22) Date of filing: 28.01.2023
(51) Int. Cl.: C07D 209/70, C07D 209/52, C07D 487/04, C07D 519/00

(54) **SYNTHESIS METHOD FOR 7,8-DIHYDRO-2H-CYCLOPENTAPYRROLOPYRAZINONE COMPOUND**

(30) Priority: 28.01.2022 CN 202210113331
(71) Applicant: HUTCHMED Limited, Shanghai 201203 (CN)
(72) Inventor: LIU, Bo, Shanghai 201203 (CN); GE, Chongfeng, Shanghai 201203 (CN); FAN, Chenguang, Shanghai 201203 (CN); WANG, Chuanguo, Shanghai 201203 (CN)
(74) Representative: Bianchetti & Minoja with Trevisan & Cuonzo IPS SRL
(86) International application number: PCT/CN2023/073516
(87) International publication number: WO 2023/143491

(57) **Abstract**

The present invention relates to a method of synthesizing a 7,8-dihydro-2*H-*cyclopenta[4,5]pyrrolo[1,2-a]pyrazine-1(6*H*)-one compound and an analog thereof. The method is well suited for large-scale industrial production because of its simple steps, simple and safe reaction operations, and easily separated and purified related intermediates.

## Description

### Technical Field

The present invention belongs to the fields of pharmaceutical intermediates and pharmaceutical chemistry. In particular, the present invention relates to a method of synthesizing a 7,8-dihydro-2*H*-cyclopenta[4,5]pyrrolo[1,2-a]pyrazin-1(6*H*)-one compound and an analog thereof.

### Background Art

7,8-dihydro-2*H*-cyclopenta[4,5]pyrrolo[1,2-a]pyrazin-1(6*H*)-one compounds and analogs thereof are a novel class of pharmaceutical intermediates.

A variety of active compounds for use in the treatment of inflammation, immune diseases and cancer, as well as intermediates for the preparation of these compounds, such as the compounds having the following structures are provided in the Chinese patent application CN 104125959 A:

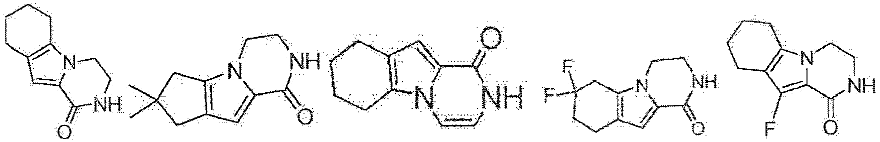

The patent application further discloses the following methods of preparing the above-mentioned compounds:

### Method 1:

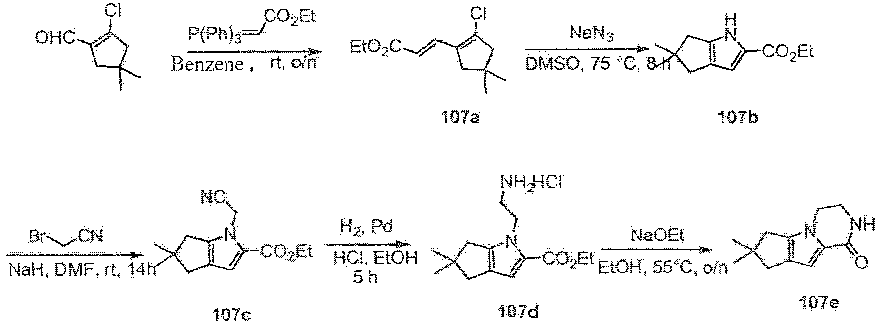

Wittig reaction is carried out in the first synthesis step of the method. The reaction is generally carried out in the presence of an aprotic solvent and a strong base and under anhydrous and oxygen-free conditions, etc., and produces an extremely unstable Wittig reagent, which is not subjected to separation and reacts directly with an aldehyde to produce compound 107a. The method is not suitable for industrial production due to the harsh conditions of the Wittig reaction. Sodium azide is used in the second step of the method to introduce an amino group (compound 107b), and due to its highly toxic and explosive nature, this route poses serious hidden safety hazards in industrial production.

### Method 2:

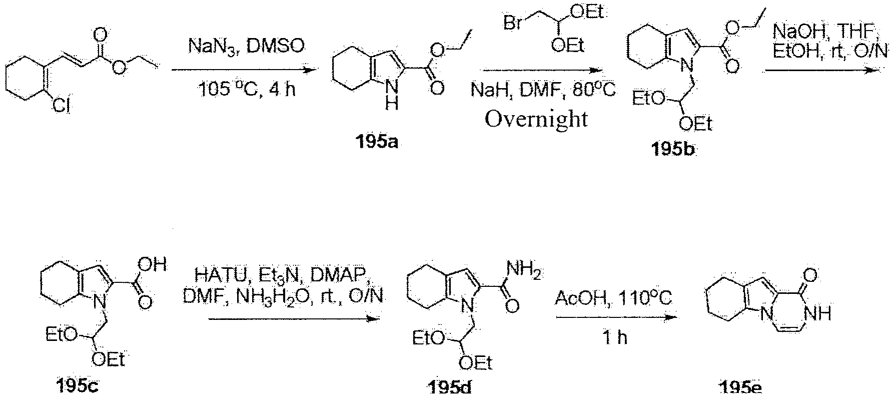

The method also uses the highly toxic and explosive reagent sodium azide, making it unsuitable for use in industrial production.

Moreover, methods 1 and 2 described above have long reaction routes, which is not conducive to industrial production.

The present invention provides a novel method of synthesizing a 7,8-dihydro-2*H-*cyclopenta[4,5]pyrrolo[1,2-a]pyrazin-1(6*H*)-one compound and an analog thereof (a compound of formula (I)). The method is well suited for large-scale industrial production because of its simple steps, simple and safe reaction operations and the related intermediates are easy to separate and purify. The present invention also provides novel intermediate compounds, which can be used for the preparation of a variety of small molecule inhibitors or intermediates thereof.

### Summary of the Invention

The present invention provides a method (hereinafter referred to as "the method of the present invention") of synthesizing a 7,8-dihydro-2*H*-cyclopenta[4,5]pyrrolo[1,2-a]pyrazin-1(6*H*)-one compound and an analog thereof (i.e., a compound as shown in formula (I)), 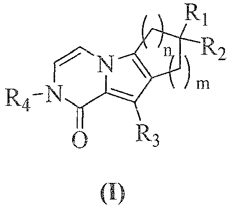
wherein: R₁ and R₂ are each independently selected from hydrogen, C₁₋₆ alkyl, or halogen;
R₃ is selected from hydrogen or C₁₋₆ alkyl;
m and n are independently 0, 1 or 2, and m + n is 2, 3 or 4; and
R₄ is selected from hydrogen or C₁₋₆ alkyl.

In one aspect, the present invention provides the following synthetic route: 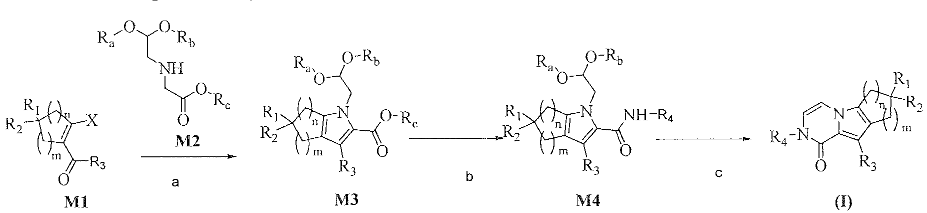 wherein the variables are as defined below.

In another aspect, the present invention also provides the following specific embodiments.

Embodiment 1: A method of synthesizing a compound of formula (I), 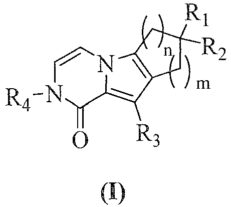
wherein: R₁ and R₂ are each independently selected from hydrogen, C₁₋₆ alkyl, or halogen;
R₃ is selected from hydrogen or C₁₋₆ alkyl;
m and n are independently 0, 1 or 2, and m + n is 2, 3 or 4; and
R₄ is selected from hydrogen or C₁₋₆ alkyl;
the method comprising the following steps:
   a) subjecting compound M1 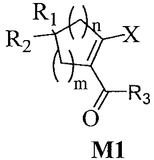
      wherein R₁, R₂, R₃, m, and n are as defined above, and X is halogen or an easily leaving active ester group such as -OTf, -OTs, or -OMs, preferably halogen,
      to a cyclization reaction with compound M2, 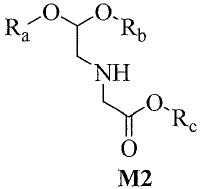
      wherein Rₐ, R_{b}, and R_{c} are each independently selected from C₁₋₆ alkyl; to obtain compound M3 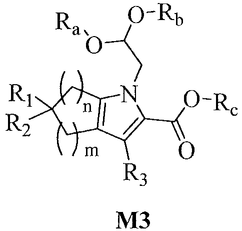
      wherein R₁, R₂, R₃, m, n, Rₐ, R_{b}, and R_{c} are as defined above;
   b) subjecting the compound M3 to an ammonolysis reaction to obtain compound M4 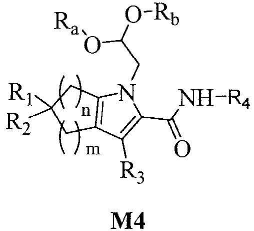 wherein R₁, R₂, R₃, R₄, m, n, Rₐ, and R_{b} are as defined above;
   c) subjecting the compound M4 to a reaction under acidic conditions to obtain the compound of formula (I).

Embodiment 2: The method according to embodiment 1, wherein step b) is replaced with step b') as follows:
subjecting the compound M3 to a hydrolysis reaction to obtain compound M5, 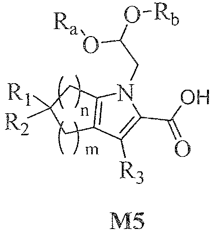
wherein R₁, R₂, R₃, m, n, Rₐ, and R_{b} are as defined above;
and then subjecting the compound M5 to a reaction with R₄NH₂ (wherein R₄ is selected from hydrogen or C₁₋₆ alkyl) to obtain the compound M4.

Embodiment 3: The method according to embodiment 1 or 2, wherein: R₁ and R₂ are each independently selected from C₁₋₆ alkyl or hydrogen, preferably methyl or hydrogen, and more preferably methyl.

Embodiment 4: The method according to any one of the preceding embodiments, wherein: R₃ is H.

Embodiment 5: The method according to any one of the preceding embodiments, wherein: Rₐ and R_{b} are each independently selected from methyl or ethyl, preferably methyl.

Embodiment 6: The method according to any one of the preceding embodiments, wherein: R_{c} is methyl or ethyl, preferably ethyl.

Embodiment 7: The method according to any one of the preceding embodiments, wherein m is 0 or 1, and n is 1 or 2; and preferably, m is 1, and n is 1.

Embodiment 8: The method according to any one of the preceding embodiments, wherein R₄ is H.

Embodiment 9: The method according to any one of the preceding embodiments, wherein: the cyclization reaction in step a) is carried out in a non-polar solvent, such as *N,N*-dimethylformamide (DMF) or N-methylpyrrolidone (NMP) or a mixed solvent thereof; and the reaction is carried out under heating conditions, preferably, at a temperature controlled at 100°C-150°C, preferably at 110°C-130°C, and more preferably at 115°C-125°C.

Embodiment 10: The method according to any one of the preceding embodiments, wherein: the ammonolysis reaction in step b) is carried out under conventional reaction conditions in the field, such as in an organic solvent methanol or ethanol, using a corresponding ammonolysis agent, such as R₄NH₂, in which R₄ is selected from hydrogen or C₁₋₆ alkyl; for example, the ammonolysis reaction can be carried out in a solution of ammonia in methanol.

Embodiment 11: The method according to any one of the preceding embodiments, wherein: the hydrolysis reaction in step b') can be carried out in the presence of a strong inorganic base, such as, in a reaction solvent (e.g., an organic solvent and water), and at 50°C-100°C (e.g., 75°C-85°C); specifically, the strong inorganic base can be selected from potassium hydroxide, sodium hydroxide, or lithium hydroxide.

Embodiment 12: The method according to any one of the preceding embodiments, wherein: the compound M5 in step b') is reacted with R₄NH₂ under alkaline conditions in the presence of an optional condensing agent, and in a solvent; preferably, the base includes, but is not limited to, triethylamine, pyridine, 4-dimethylaminopyridine, DBU, etc.; and preferably, the condensing agent includes, but is not limited to, HBTU, DCC, EDCI, DIC, or CDI.

Embodiment 13: The method according to any one of the preceding embodiments, wherein: the acid in step c) is selected from an organic acid, such as acetic acid, p-toluenesulfonic acid, etc.; and preferably, the reaction is carried out at a temperature controlled at 70°C-120°C, such as 95°C-105°C.

Embodiment 14: A method of preparing compound M3, 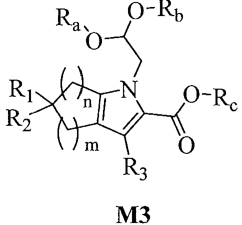
wherein R₁, R₂, R₃, m, n, Rₐ, R_{b}, and R_{c} are as defined above;
the method comprising the following steps:
   subjecting compound M1 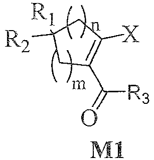
   wherein R₁, R₂, R₃, X, m, and n are as defined above,
   to a cyclization reaction with compound M2, 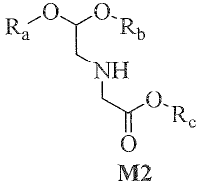
   wherein Rₐ, R_{b}, and R_{c} are as defined above;
   to obtain the compound M3.

Embodiment 15: The method according to embodiment 14, wherein the cyclization reaction is carried out according to Embodiment 9.

Embodiment 16: A compound or a salt thereof, wherein the compound is selected from 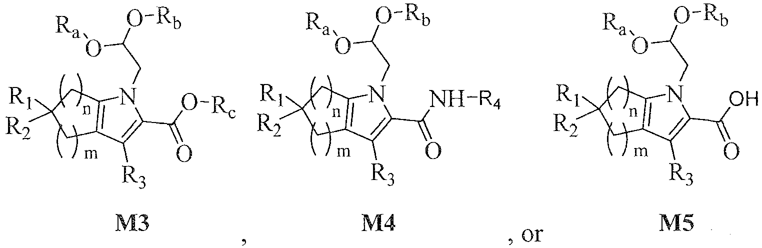 wherein R₁, R₂, R₃, R₄, m, n, Rₐ, R_{b}, and R_{c} are as defined above.

Embodiment 17: A compound or a salt thereof, wherein the compound is selected from

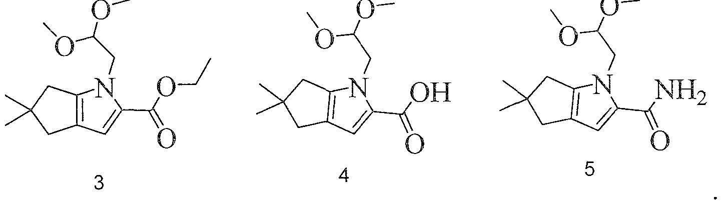

Embodiment 18: Use of the compound or the salt thereof according to Embodiment 16 or 17 in the preparation of a compound of formula (I).

### Definitions:

The terms or symbols used in the present application have the meanings as set forth below, unless otherwise specified. Technical and scientific terms as used herein and not specifically defined have the meaning commonly understood by the POSITA to which the present invention belongs.

The term "alkyl" as used herein refers to a linear or branched saturated monovalent hydrocarbyl having 1 to 6 carbon atoms, such as 1, 2, 3, 4, 5, or 6 carbon atoms, preferably a linear or branched saturated monovalent hydrocarbyl having 1 to 4 carbon atoms. The alkyl having 1 to 6 carbon atoms is simply represented as "C₁₋₆ alkyl", the alkyl having 1 to 4 carbon atoms is simply represented as "C₁₋₄ alkyl", and the alkyl having other numbers of carbon atoms can also be represented in a similar manner. Examples of the alkyl include, but are not limited to: methyl, ethyl, *n*-propyl, isopropyl, *n*-butyl, isobutyl, *tert*-butyl, and the like.

The term "halogen" or "halo" as used herein means fluorine (F), chlorine (Cl), bromine (Br), and iodine (I), preferably chlorine and bromine, and more preferably chlorine.

The term "strong inorganic base" as used in step b') herein refers to an inorganic base that is capable of hydrolyzing a carboxylic ester to produce a carboxylate or carboxylic acid, including but not limited to potassium hydroxide, sodium hydroxide, or lithium hydroxide.

The term "easily leaving active ester group" as used herein refers to an ester group prepared from an alcohol, which can be easily replaced by a nucleophile, such as an amine, to form a stable linkage. The term "easily leaving active ester group" of the present invention is preferably p-CH₃-Ph-SO₃-(-OTs), CF₃SO₃-(-OTf) or CH₃SO₃- (-OMs), wherein Ph refers to phenyl, Ts refers to p-toluenesulfonyl, Tf refers to trifluoromethanesulfonyl, and Ms refers to methylsulfonyl.

The term "condensing agent" as used herein refers to commonly used condensing agents that can facilitate the formation of amides, including but not limited to HBTU, DCC, EDCI, DIC, or CDI.
DBU refers to 1,8-diazabicyclo[5.4.0]undec-7-ene.
HBTU refers to benzotriazole-N,N,N',N'-tetramethyluronium hexafluorophosphate.
DCC refers to N,N'-dicyclohexylcarbodiimide.
EDCI refers to 1-(3-dimethylaminopropyl)-3-ethylcarbodiimide hydrochloride.
DIC refers to N,N'-diisopropylcarbodiimide.
CDI refers to N,N'-carbonyldiimidazole.

It should be understood that when a later embodiment or technical solution refers to a prior embodiment or technical solution and does not further define the variables or features described therein, the variables or features in the later embodiment or technical solution have the same meaning or definition as the corresponding variables or features of the prior embodiment or technical solution, unless otherwise specified or clearly contradicted by context.

It should also be understood that the reactions of the present invention can be carried out under conventional reaction conditions known in the field. For example, the reaction temperature and pressure can be controlled to a certain extent according to the characteristics of the reactants until the reaction is completed.

Generally, the reactions can be carried out in a solvent, including, but not limited to, organic solvents such as methanol, ethanol, isopropanol, acetonitrile, heptane, toluene, acetone, dichloromethane, tetrahydrofuran, 2-methyloxolane, dimethyl sulfoxide, N,N-dimethylformamide, ethyl acetate, ethyl ether, isopropyl ether, methyl tert-butyl ether, methyl cyclopentyl ether, and the like, as well as any combination thereof. Where appropriate, a mixed solvent of an organic solvent and water can be used. Some reactions can be carried out without additional solvents, and the reaction reagents themselves can be used as solvents. For example, the acid used in step c) such as acetic acid, can be used as a solvent, or other solvents can be added to the reaction.

The reactions can be carried out at appropriate temperatures, for example at -78°C to 200°C, such as -78°C-0°C, -20°C-20°C, -10°C-10°C, 10°C-130°C, 20°C-100°C, 40°C-100°C, 50°C-100°C, 60°C-100°C, 50°C-80°C, 70°C-120°C, 60°C-90°C, 110°C-130°C, 110°C-120°C, 100°C-150°C, or 100°C-200°C.

The reaction can be carried out under normal pressure, elevated pressure, or reduced pressure, as appropriate.

It should be understood that the POSITA can easily detect the reaction process and thereby determine the reaction time or the end point of the reaction according to the actual situation.

Herein, in the case of inconsistency of the name and structure of a compound, when the two of which are both given for the compound, it is subject to the structure of the compound, unless the context shows that the structure of the compound is incorrect, and the name is correct.

The process route for synthesizing the compound of formula (I) provided by the present invention has the following beneficial effects:
1. There are only three steps of reaction in the entire process route, each with a single reaction site and very few side reactions;
2. The reaction conditions are mild and the operation is simple, without hazardous reagents, such as highly toxic reagents and flammable and explosive reagents, making the process route well suited for industrial production;
3. The production process is simple and the cost is low; and/or
4. The related intermediates are easy to separate and purify, and can even be used directly in the subsequent reaction without purification, making the process route well suited for large-scale industrial production.

### Detailed Description of Embodiments

The examples below are intended to be purely exemplary and should not be considered to be limiting in any way. Efforts have been made to ensure the accuracy with respect to numbers used (for example, amounts, temperature, etc.), but those skilled in the art should understand that some experimental errors and deviations should be accounted for. Unless indicated otherwise, parts are parts by weight, temperatures in degrees Centigrade, and the pressure is at or near atmospheric. All HNMR data were generated using Varian 400-MR. All reagents used in the examples of the present invention are commercially available.

### Example 1

### Synthesis of ethyl 1-(2,2-dimethoxyethyl)-5,5-dimethyl-1,4,5,6-tetrahydrocyclopenta [b] pyrrole-2-carboxylate

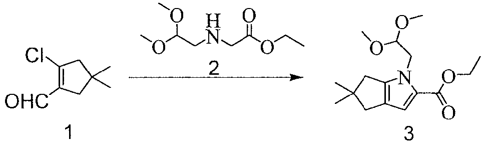

To a 2000 L reaction kettle were added compound 1 (50.0 kg), compound 2 (150.0 kg), and DMF (*N,N*-dimethylformamide, 950 kg). The mixture was stirred and heated and the temperature was controlled at 115°C-125°C until the reaction was completed. The reaction liquid was cooled to room temperature and then added to water. Ethyl acetate was added, and the resulting mixture was stirred and then left to stand for liquid separation. The organic phase was collected and washed with saturated aqueous NaCl solution and left to stand for liquid separation. The organic phase was collected. The organic phase was concentrated under reduced pressure until no fraction was distilled off.

A mixed solvent of *n*-heptane/ethyl acetate was added to dissolve the distillation residue, and the solution was filtered through a silica gel pad to remove a small amount of insoluble matter. The filter cake was washed with a mixed solvent of n-heptane/ethyl acetate. The filtrates were combined. The filtrate was concentrated under reduced pressure until no obvious liquid dripped, to obtain a pale yellow oil 42.8 kg, (compound 3, ethyl 1-(2,2-dimethoxyethyl)-5,5-dimethyl-1,4,5,6-tetrahydrocyclopenta[b]pyrrole-2-carboxylate) (content: 57.2%, yield: 26.3%, MS (m/z) = 264.0 [M-OMe]⁺). The product was used directly in the next step without purification.

A small amount of the sample was taken for purification and the HNMR data measured for the product were as follows:
¹HNMR (400MHz, CDCl₃) δ 6.73 (s, 1H), 4.54 (t, *J* = 5.3Hz, 1H), 4.28 - 4.18 (m, 4H), 3.35 (s, 6H), 2.54 (s, 2H), 2.42 (s, 2H), 1.32 (t, *J* = 7.1Hz, 3H), 1.20 (s, 6H).

### Example 2

### Synthesis of 1-(2,2-dimethoxyethyl)-5,5-dimethyl-1,4,5,6-tetrahydrocyclopenta[b]pyrrole-2-carboxylic acid

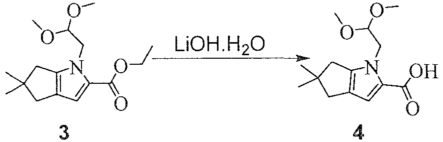

To a 500 L reaction kettle were added ethanol (99.0 kg), water (125.0 kg), compound 3 (25.0 kg), and LiOH.H₂O (14.2 kg). The mixture was heated to 75°C-85°C and stirred while the temperature was maintained constant until the reaction was completed. The reaction liquid was concentrated under reduced pressure to remove most of the ethanol. Methyl *tert*-butyl ether was added, and the resulting mixture was stirred and then left to stand for liquid separation. The lower aqueous phase was collected, and the aqueous phase was adjusted to pH 5-6 with aqueous hydrochloric acid solution and a large amount of solid was precipitated. The reaction mixture was filtered, and the filter cake was washed with water and dried to obtain an off-white solid 19.8 kg, (compound 4, 1-(2,2-dimethoxyethyl)-5,5-dimethyl-1,4,5,6-tetrahydrocyclopenta[b]pyrrole-2-carboxylic acid) (purity: 91.97%, content: 91%, yield: 79.6%, MS (m/z) = 236.0 [M-OMe]⁺).

The HNMR data of the product were as follows:
¹HNMR (400MHz, CDCl₃) δ 6.86 (s, 1H), 4.55 (t, *J =* 5.3Hz, 1H), 4.21 (d, *J =* 5.3Hz, 2H), 3.36 (s, 6H), 2.56 (s, 2H), 2.44 (s, 2H), 1.21 (s, 6H).

### Example 3

### Synthesis of 1-(2,2-dimethoxyethyl)-5,5-dimethyl-1,4,5,6-tetrahydrocyclopenta [b] pyrrole-2-carboxamide

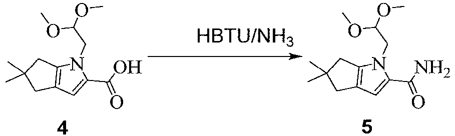

To a reaction kettle were added DMF (102.6 kg), compound 4 (19.8 kg), and triethylamine (13.6 kg). The mixture was cooled to -5°C to 5°C. HBTU (benzotriazole-*N,N,N',N'*-tetramethyluronium hexafluorophosphate, 30.6 kg) was added in batches at a controlled temperature of less than 10°C. After the addition, the mixture was stirred at a constant temperature of -5°C to 5°C until the reaction of compound 4 was completed. Ammonia water was added dropwise to the reaction liquid at a controlled temperature of less than 10°C, and the resulting mixture was stirred at a constant temperature of -5°C to 5°C until the reaction was completed. The reaction liquid was poured into water, and ethyl acetate was added. The mixture was stirred and then left to stand for liquid separation. The organic phase was collected and washed with saturated aqueous NaCl solution and left to stand for liquid separation. The organic phase was collected. The organic phase was concentrated under reduced pressure and distilled until no obvious liquid dripped, to obtain a light red solid 18.6 kg, (compound 5, 1-(2,2-dimethoxyethyl)-5,5-dimethyl-1,4,5,6-tetrahydrocyclopenta[b]pyrrole-2-carboxamide) (purity: 94.6%, content: 94.1%, yield: 97.8%, MS (m/z) = 235.1 [M-OMe]⁺). The product was used directly in the next step without purification.

A small amount of the sample was taken for purification and the HNMR data measured for the product were as follows:
¹HNMR (400MHz, CDCl₃) δ 6.40 (s, 1H), 5.42 (s, 2H), 4.61 (t, *J =* 5.2Hz, 1H), 4.23 (d, *J* = 5.2Hz, 2H), 3.36 (s, 6H), 2.55 (s, 2H), 2.42 (s, 2H), 1.20 (s, 6H).

### Example 4

### 7,7-dimethyl-7,8-dihydro-2H-cyclopenta[4,5]pyrrolo[1,2-a]pyrazin-1(6H)-one

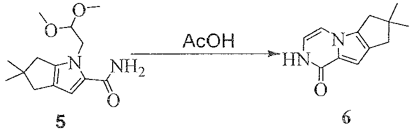

The intermediate compound 5 (17.5 kg) obtained in Example 3 was dissolved with acetic acid (147 kg) and then added to a 200 L reaction kettle. The temperature was controlled at 95°C-105°C until the reaction was completed. The reaction solution was cooled to room temperature and then poured into water. A mixed solvent of ethyl acetate and ethanol was added under stirring. A mixed solution of the reaction liquid and water was extracted and then left to stand for liquid separation. The organic phases were combined. The organic phase was concentrated under reduced pressure to a small volume and a large amount of solid was precipitated. To the reaction kettle were added *n*-heptane and ethyl acetate. The resulting mixture was heated to 40°C-50°C and stirred while the temperature was maintained constant. The reaction mixture was subjected to hot filtration and the filter cake was washed with a mixed solvent of *n*-heptane and ethyl acetate and dried to obtain an off-white solid 11.1 kg, (compound 6, 7,7-dimethyl-7,8-dihydro-2H-cyclopenta[4,5]pyrrolo[1,2-a]pyrazin-1(6H)-one) (purity: 99.8%, content: 98.4%, yield: 83.5%, MS (m/z) = 203.0 [M+H]⁺).

The HNMR data of the product were as follows:
¹HNMR (400MHz, CDCl₃) δ 10.92 (s, 1H), 6.89 (s, 1H), 6.74 (d, *J =* 5.6Hz, 1H), 6.51 (t, *J* = 4.5Hz, 1H), 2.63 (s, 2H), 2.60 (s, 2H), 1.26 (s, 6H).

## Claims

1. A method of synthesizing a compound of formula (I), 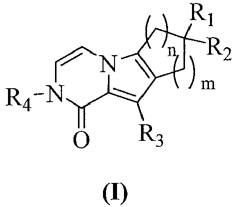
wherein: R₁ and R₂ are each independently selected from hydrogen, C₁₋₆ alkyl, or halogen;
R₃ is selected from hydrogen or C₁₋₆ alkyl;
m and n are independently 0, 1 or 2, and m + n is 2, 3 or 4; and
R₄ is selected from hydrogen or C₁₋₆ alkyl;
the method comprising the following steps:
a) subjecting compound M1 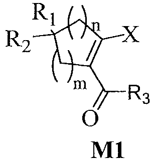
wherein R₁, R₂, R₃, m, and n are as defined above, and X is halogen or an easily leaving active ester group such as -OTf, -OTs, or -OMs, preferably halogen,
to a cyclization reaction with compound M2, 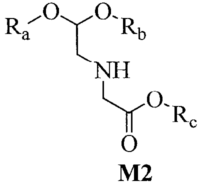
wherein Rₐ, R_{b}, and R_{c} are each independently selected from C₁₋₆ alkyl; to obtain compound M3 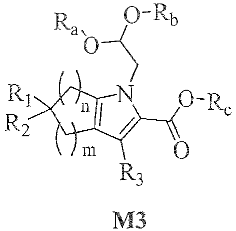
wherein R₁, R₂, R₃, m, n, Rₐ, R_{b}, and R_{c} are as defined above;
b) subjecting the compound M3 to an ammonolysis reaction to obtain compound M4 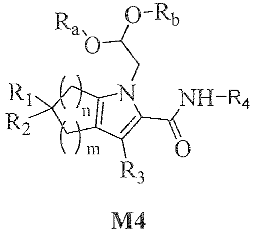 wherein R₁, R₂, R₃, R₄, m, n, Rₐ, and R_{b} are as defined above;
c) subjecting the compound M4 to a reaction under acidic conditions to obtain the compound of formula (I).

2. The method according to claim 1, wherein step b) is replaced with step b') as follows:
subjecting the compound M3 to a hydrolysis reaction to obtain compound M5, 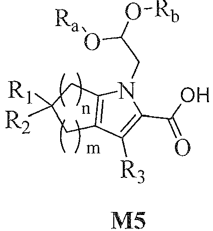
wherein R₁, R₂, R₃, m, n, Rₐ, and R_{b} are as defined in claim 1;
and then subjecting the compound M5 to a reaction with R₄NH₂, in which R₄ is selected from hydrogen or C₁₋₆ alkyl, to obtain the compound M4.

3. The method according to claim 1 or 2, wherein: R₁ and R₂ are each independently selected from C₁₋₆ alkyl or hydrogen, preferably methyl or hydrogen, and more preferably methyl.

4. The method according to any one of the preceding claims, wherein: R₃ is H.

5. The method according to any one of the preceding claims, wherein: Rₐ and R_{b} are each independently selected from methyl or ethyl, preferably methyl.

6. The method according to any one of the preceding claims, wherein: R_{c} is methyl or ethyl, preferably ethyl.

7. The method according to any one of the preceding claims, wherein m is 0 or 1, and n is 1 or 2; and preferably, m is 1, and n is 1.

8. The method according to any one of the preceding claims, wherein R₄ is H.

9. The method according to any one of the preceding claims, wherein: the cyclization reaction in step a) is carried out in a non-polar solvent, such as *N,N-*dimethylformamide (DMF) or N-methylpyrrolidone (NMP) or a mixed solvent thereof; and the reaction is carried out under heating conditions, preferably, at a temperature controlled at 100°C-150°C, preferably at 110°C-130°C, and more preferably at 115°C-125°C.

10. The method according to any one of the preceding claims, wherein: the ammonolysis reaction in step b) is carried out under conventional reaction conditions in the filed such as in an organic solvent methanol or ethanol, using a corresponding ammonolysis agent such as R₄NH₂, in which R₄ is selected from hydrogen or C₁₋₆ alkyl; for example, the ammonolysis reaction can be carried out in a solution of ammonia in methanol.

11. The method according to any one of the preceding claims, wherein: the hydrolysis reaction in step b') can be carried out in the presence of a strong inorganic base, such as, in a reaction solvent, e.g., an organic solvent and water, and at 50°C-100°C; specifically, the strong inorganic base can be selected from potassium hydroxide, sodium hydroxide, or lithium hydroxide.

12. The method according to any one of the preceding claims, wherein: the compound M5 in step b') is reacted with R₄NH₂ under alkaline conditions in the presence of an optional condensing agent, and in a solvent; preferably, the base includes, but is not limited to, triethylamine, pyridine, 4-dimethylaminopyridine, DBU, etc.; and preferably, the condensing agent includes, but is not limited to, HBTU, DCC, EDCI, DIC, or CDI.

13. The method according to any one of the preceding claims, wherein: the acid in step c) is selected from an organic acid such as acetic acid, *p*-toluenesulfonic acid, etc.; and preferably, the reaction is carried out at a temperature controlled at 70°C-120°C, such as 95°C-105°C.

14. A method of preparing compound M3, 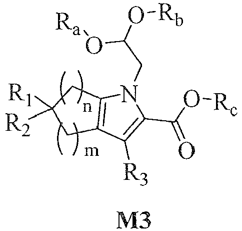
wherein R₁, R₂, R₃, m, n, Rₐ, R_{b}, and R_{c} are as defined in claim 1;
the method comprising the following steps:
subjecting compound M1 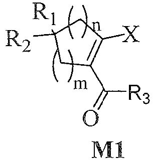
wherein R₁, R₂, R₃, X, m, and n are as defined in claim 1,
to a cyclization reaction with compound M2, 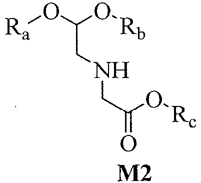
wherein Rₐ, R_{b}, and R_{c} are as defined in claim 1;
to obtain the compound M3.

15. The method according to claim 14, wherein the cyclization reaction is carried out according to claim 9.

16. A compound or a salt thereof, wherein the compound is selected from 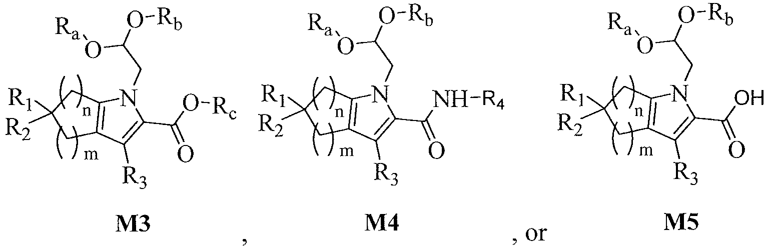 wherein R₁, R₂, R₃, R₄, m, n, Rₐ, R_{b}, and R_{c} are as defined in claim 1.

17. A compound or a salt thereof, wherein the compound is selected from

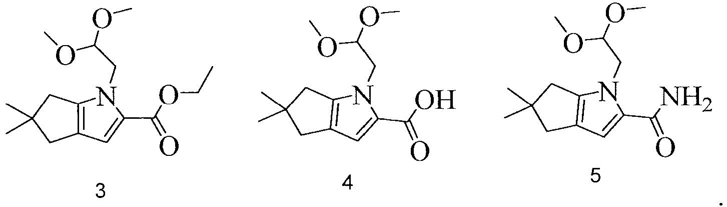

18. Use of the compound or the salt thereof according to claim 16 or 17 in the preparation of the compound of formula (I) according to claim 1.
